# EUROPÄISCHE PATENTSCHRIFT

(11) **EP 1 853 218 B1**
(45) Veröffentlichungstag und Bekanntmachung des Hinweises auf die Patenterteilung: **03.07.2013**
(21) Anmeldenummer: 06706868.4
(22) Anmeldetag: 10.02.2006
(51) Int. Cl.: A61K 9/00, A61K 9/28, A61K 31/675

(54) **TROFOSFAMID-HALTIGE FILMTABLETTEN UND VERFAHREN ZU DEREN HERSTELLUNG**
TROFOSFAMIDE-CONTAINING FILM-COATED TABLETS AND METHOD FOR THE PRODUCTION THEREOF
COMPRIMES PELLICULAIRES A BASE DE TROFOSFAMIDE ET PROCEDE PERMETTANT DE LES PRODUIRE

(30) Priorität: 25.02.2005 DE 102005008797
(43) Veröffentlichungstag der Anmeldung: 14.11.2007
(73) Patentinhaber: Baxter International Inc., Deerfield, IL 60015 (US); Baxter Healthcare SA, 8152 Glattpark (Opfikon) (CH)
(72) Erfinder: ROESSLER, Berthold, 33790 Halle/Westfalen (DE)
(74) Vertreter: Müller-Boré & Partner Patentanwälte
(86) Internationale Anmeldenummer: PCT/EP2006/001248
(87) Internationale Veröffentlichungsnummer: WO 2006/089651

(56) Entgegenhaltungen:
- WO-A-99/65499
- WO-A-2005/049091
- DE-A1- 3 804 686
- DE-A1- 4 244 466
- US-A- 4 218 471
- US-A- 5 047 246
- US-A- 5 158 776
- HEMPEL GEORG ET AL: "Pharmacokinetics of trofosfamide and its dechloroethylated metabolites" CANCER CHEMOTHERAPY AND PHARMACOLOGY, Bd. 40, Nr. 1, 1997, Seiten 45-50, XP002404909 ISSN: 0344-5704

## Beschreibung

Die vorliegende Erfindung betrifft Trofosfamid enthaltende Filmtabletten zur oralen Verabreichung und ein Verfahren zu deren Herstellung.

Trofosfamid (3-(2-chlorethyl)-2-[bis-(2-chlorethyl)-amino]-tetrahydro-2H-1,3,2-oxazaphosphorin-2-oxid) ist ein Prodrug, das im Körper in die aktiven Formen der alkylierenden Zytostatika Ifosfamid und Cyclofosfamid umgewandelt wird. Bei der Umwandlung des Trofosfamids entstehen die Abbauprodukte in einem Verhältnis von 6:1 (Ifosfamid : Cyclofosfamid).

Beide Abbauprodukte des Trofosfamids sind in der medikamentösen Therapie von Tumorerkrankungen gut bekannte und hinlänglich erprobte Substanzen. Der zytotoxische Wirkstoff Trofosfamid wird derzeit in der medikamentösen Therapie von lymphoretikulären Tumoren und Hämoblastosen verwendet. Hierzu wird es täglich oral in einer Dosierung von 50 mg bis 150 mg in der Erhaltungstherapie verabreicht.

Trofosfamid ("Ixoten") wird insbesondere in der Therapie des Patienten zu Hause eingesetzt. Dies erfordert, dass das Umfeld des Patienten vor einer eventuellen Kontamination durch den zytotoxischen Wirkstoff besonders geschützt wird. Zu diesem Zweck muss eine orale Darreichungsform von Trofosfamid auf geeignete Weise ummantelt oder überzogen sein, damit kein Wirkstoff aus der Verpackung unkontrolliert freigesetzt wird.

Übliche Methoden des Ummantelns oder Überziehens sind die Herstellung von Manteltabletten, Dragees oder Filmtabletten [siehe z.B. R. Voigt, Pharmazeutische Technologie]. Die Art des Überzuges oder Mantels richtet sich nach den Eigenschaften des Wirkstoffes und seiner Empfindlichkeit gegenüber dem Herstellungsverfahren der entsprechenden Darreichungsform. Hydrolyseempfindliche Wirkstoffe wie Trofosfamid lassen sich üblicherweise nicht dragieren (Verwendung von warmen, wässrigem Zuckersirup) oder mit wässrigen Filmsuspensionen überziehen, da sich der Wirkstoff zersetzt.

Aufgrund der Hydrolyseempfindlichkeit von Trofosfamid ist derzeit daher nur eine Manteltablette als Darreichungsform bekannt, in welcher der wirkstoffhaltige Kern von einem Mantel aus Tablettierhilfsstoffen umgeben ist. Technisch wird dies derart realisiert, dass zunächst der Tablettenkern mit wenig Hilfsstoffen auf einer Tablettenmaschine gepresst wird und dieser Kern anschließend erneut verpresst wird, wobei ein Bett von Hilfsstoffen um ihn herum gepresst wird. Im Ergebnis ist durch dieses "trockene Ummanteln" der Wirkstoff im Kern komplett von einem Hilfsstoffmantel umgeben. Dieser Hilfsstoffmantel bewahrt den Hydrolyseempfindlichen Wirkstoff vor Feuchtigkeit, um eine Zersetzung zu verhindern, und schützt gleichzeitig den Patienten und dessen Umgebung vor einer Kontamination mit dem hochpotenten Wirkstoff. Die Herstellung einer Trofosfamid-enthaltenden Filmtablette ist bisher im Stand der Technik aufgrund der Hydrolyseempfindlichkeit dieses Wirkstoffes nicht beschrieben.
US 4,218,471 beschreibt ein Verfahren zur Behandlung von urotoxischen Nebenwirkungen bei Patienten, die mit cytostatischen Alkylierungsmitteln behandelt werden. Dieses Verfahren verwendet *inter alia* ummantelte Tabletten, die Trofosfamid enthalten. Die Ummantelung wird dabei aus einem trockenen Granulat hergestellt.
Hempel et al, Cancer Chemotherapy and Pharmacology, Bd. 40, Nr. 1, 1997, Seiten 45-50, untersucht die Pharmakokinetik von Trofosfamid und dessen Metaboliten. Dabei wird Trofosfamid oral verabreicht.
US 5,158,776 bzw. EP 0 469 440 beschreiben Ifosfamid enthaltende Kapseln und Tabletten. Die Tabletten können in einem Sprühverfahren mit geeigneten wäßrigen Dispersionen beschichtet werden.
WO 99/65499 beschreibt Cyclophosphamid enthaltende Filmtabletten. Diese werden in einem Sprühverfahren mit einer wäßrigen Suspension beschichtet.
US 5,047,246 beschreibt ummantelte Cyclophosphamid enthaltende Tabletten. Die Ummantelung wird dabei aus einem trockenen Granulat hergestellt.
DE 42 44 466 beschreibt ein Verfahren zur Herstellung von Pseudolatices und Mikro- oder Nanopartikeln, die sich *inter alia* zur Herstellung von Diffusionsfilmen verwenden lassen, welche wiederum für die Herstellung von Filmtabletten verwendet werden können. Des weiteren wird ein Verfahren zur Herstellung von wirkstoffhaltigen Mikropartikeln beschrieben, die z.B. auch Cyclophosphamid enthalten können.

Somit liegt der vorliegenden Erfindung die Aufgabe zugrunde, Trofosfamid-haltige Filmtabletten sowie ein Verfahren zur Herstellung derartiger Filmtabletten bereitzustellen.

Diese Aufgabe wird durch die in den Ansprüchen gekennzeichneten Ausführungsformen gelöst.

Insbesondere wird eine Filmtablette bereitgestellt, welche einen Trofosfamid enthaltenden Kern und einen Film bzw. Überzug umfasst, wobei dieser Film durch Aufbringen einer wässrigen Suspension, welche eine oder mehrere Filmbildner enthält, auf den Kern erhältlich ist. Der Kern der erfindungsgemäßen Filmtablette weist vorzugsweise etwa 20 mg bis etwa 200 mg, mehr bevorzugt etwa 50 mg bis 100 mg, besonders bevorzugt etwa 50 mg Trofosfamid als pharmazeutisch wirksamen Bestandteil je Filmtablette auf. Die Gesamtmasse der erfindungsgemäßen Filmtablette beträgt vorzugsweise etwa 100 mg bis 400 mg, mehr bevorzugt etwa 100 mg bis etwa 200 mg, besonders bevorzugt 140 bis 170 mg, wobei die Gesamtmasse der Filmtablette vorzugsweise in Abhängigkeit vom Anteil des Trofosfamids eingestellt wird. Der Anteil des Films liegt vorzugsweise in einem Bereich von etwa 1 % bis etwa 15%, mehr bevorzugt etwa 2% bis etwa 10%, besonders bevorzugt etwa 3% bis etwa 6%, bezogen auf die Gesamtmasse der erfindungsgemäßen Filmtablette.

Der Anteil des einen oder der mehreren Filmbildner liegt in einem Bereich von etwa 30% bis etwa 70%, bezogen auf die Gesamtmasse des Films. Der erfindungsgemäß verwendete Filmbildner unterliegt keiner besonderen Einschränkung, solange er pharmazeutisch verträglich ist. In einer bevorzugten Ausführungsform ist der Filmbildner ein synthetisches oder teilsynthetisches Polymer, wobei Cellulosederivate wie Hypromellose, Carboxymethylcellulose, Hydroxyethylcellulose oder Hydroxymethylcellulose, Polyvinylderivate wie Polyvinylacetatphthalat oder Polyvinylpyrrolidon, Poly(meth)acrylsäurederivate wie Eudragit NE, Eudragit E oder Eudragit L30, Cellulosephthalate wie Hydroxypropylmethylcellulosephthalate, und Shelllack beispielhaft aufgeführt werden können.

Der Trofosfamid enthaltende Kern kann im Stand der Technik bekannte, pharmazeutisch verträgliche Hilfsstoffe und/oder Trägerstoffe, wie beispielsweise herkömmliche Bindemittel, Sprengmittel und/oder Gleitmittel in den für die Tablettierung üblichen Gewichtsanteilen enthalten. Die einen oder mehrere Filmbildner enthaltende wässrige Suspension zur Herstellung des Films kann ebenfalls im Stand der Technik bekannte, pharmazeutisch verträgliche Hilfsstoffe wie Polyethylenglycole, Titandioxid, Entschäumer (z.B. Dimeticon) oder Tenside (z.B. Polysorbate) enthalten.

Ein weiterer Gegenstand der vorliegenden Erfindung betrifft ein Verfahren zur Herstellung einer vorstehend definierten Filmtablette, worin die Filmung bzw. das Überziehen bzw. das Beschichten des Trofosfamid enthaltenden Kerns durch Aufsprühen einer wässrigen Suspension, welche einen oder mehrere, wie vorstehend definierten Filmbildner enthält, bei einer Temperatur von etwa 20°C bis etwa 40°C, bevorzugt bei etwa 22°C bis etwa 35°C, besonders bevorzugt bei etwa 24°C bis etwa 30°C erfolgt. Die Sprühflussrate zum Aufbringen der Filmsuspension kann vorzugsweise im Bereich von etwa 5 bis etwa 25 ml/min, mehr bevorzugt von etwa 10 bis etwa 20 ml/min, je 4,5 kg Tablettenkerne betragen.

Die Filmung des Trofosfamid enthaltenden Kerns bzw. das Aufbringen der vorstehenden definierten wässrigen Suspension auf den Kern durch Sprühung unterliegt hinsichtlich der dafür zu verwendenden Vorrichtung keiner besonderen Einschränkung und kann beispielsweise in einem Dragierkessel erfolgen. Ferner können auch andere in der Stand der Technik bekannte Sprühbeschichtungsverfahren wie z.B. das Wirbelschichtverfahren, das Accela-cota-Verfahren oder das Glatt-Coater-Verfahren durch geeignete Anpassung an das erfindungsgemäße Verfahren verwendet werden.

Mit dem erfindungsgemäßen Verfahren ist es überraschenderweise möglich, eine hydrolytische Zersetzung des Wirkstoffes Trofosfamid aufgrund eines Kontakts mit der wässrigen Suspension durch Einhalten der vorstehend aufgeführten Prozessparameter zu verhindern. Die Einhaltung dieser Prozessparameter bzw. eine darauf basierende Prozesssteuerung ist zudem von besonderer Bedeutung, als Trofosfamid einen relativ niedrigen Schmelzpunkt von 51 °C aufweist und ab etwa 40°C zum Sintern neigt. Daher ist ein beliebiges Aufheizen des Tablettenkernbettes, beispielsweise in einem Dragierkessel zum schnellen Abtrocknen des Wassers aus der Filmsuspension, nicht möglich.

In einer erfindungsgemäßen Ausführungsform zur Herstellung der Trofosfamidhaltigen Filmtabletten werden zunächst Tablettenkerne mit einer herkömmlichen Rundläufer-Tablettenpresse gefertigt. Dabei finden übliche Tablettierhilfstoffe wie Bindemittel, Sprengmittel und Gleitmittel Verwendung. Die so erhaltenen Tablettenkerne werden in einem Dragierkessel überführt, wobei die durch die Größe des Kessels bedingten Kernmengen berücksichtigt werden sollten. Das Kernbett im Dragierkessel wird durch ständiges Rotieren des Kessels kontinuierlich in Bewegung gehalten, während erwärmte Zuluft von etwa 20 bis etwa 30°C, vorzugsweise von etwa 25 bis etwa 28°C, in das Kernbett eingeblasen wird. Bei Erreichen einer Kernbett-Temperatur von etwa 20 bis etwa 28°C kann die wässrige, einen oder mehrere Filmbildner enthaltende Suspension über eine Düse eingesprüht werden, wobei dies kontinuierlich mit einer Flussrate von beispielsweise 10 bis 20 ml/min erfolgen kann. Die erfindungsgemäß verwendete Düse, die Sprühflussrate und der Druck in dem Dragierkessel sind in an sich bekannter Weise der Menge an Tablettenkernen anzupassen. Die erwärmte Zuluft trocknet unter diesen Bedingungen das überschüssige Wasser der auf den Kern aufzubringenden sowie bereits aufgebrachten Suspension rasch ab, so dass der hydrolytisch instabile Wirkstoff Trofosfamid nur für eine kurze Zeit mit Wasser in Kontakt kommen kann und damit eine Zersetzung des Wirkstoffs verhindert wird.

Die nachfolgenden Beispiele dienen der weiteren Erläuterung der vorliegenden Erfindung, ohne einschränkend zu sein. Die in den Beispielen 1 und 3 angegebenen Parameter und Rezepturen basieren auf einer Ansatzgröße von 30.000 Tablettenkernen.

### Beispiel 1: Trofosfamid-Filmtablette mit einem Cellulosederivat-haltigen Film

Eine Trofosfamid-Filmtablette setzt sich wie folgt zusammen:

| **Inhaltsstoff** | **Menge** |
|---|---|
| **Tablettenkern** | |
| Trosfosfamid | 50,000 mg |
| Lactose Monohydrat (Lactose D10) | 50,000 mg |
| Microcrystalline Cellulose (Avicel PH 101) | 30,000 mg |
| Maisstärke | 10,000 mg |
| Talkum | 9,000 mg |
| Colloidal Siliciumdioxid (Aerosil 200 V) | 1,000 mg |
| Σ | 150,000 mg |

| **Film** | |
|---|---|
| Hypromellose (Pharmacoat) | 3,428 mg |
| Polyethylenglycol 6000 | 1,429 mg |
| Titandioxid (Pretiox) | 0,971 mg |
| Sicovit Yellow 10 E172 | 0,143 mg |
| Dimeticon (Silfar SE4) | 0,029 mg |
| Σ | 6,000 mg |

| **Filmtablette** | |
|---|---|
| Σ | 156,000 mg |

Die unter "Tablettenkern" aufgeführten Bestandteile werden gesiebt und homogen gemischt; hierzu können gängige Mischersysteme verwendet werden. Anschließend wird die Pulvermischung auf einer Rundläuferpresse zu Tabletten von ca. 150 mg Gesamtgewicht verpresst.

Die Tablettenkerne werden in einen Dragierkessel überführt und unter stetigem Rotieren mit Warmluft auf etwa 25°C temperiert. Über eine Düse wird kontinuierlich die Filmsuspension mit den unter "Film" aufgeführten Bestandteilen auf die Tablettenkerne aufgesprüht, wobei durch die Warmluft zugleich das Lösungsmittel verdunstet wird.

Die Prozessparameter für einen Ansatz von 30.000 Filmtabletten sind in Beispiel 3 wiedergegeben.

### Beispiel 2: Trofosfamid-Filmtablette mit einem Polyacrylatderivat-haltigen Film

Die Herstellung von Trofosfamid-Filmtabletten ist auch unter Verwendung von substituierten Polyacrylaten, wie Eudragit NE, möglich. Die Zusammensetzung des Tablettenkerns ist dabei unverändert wie in Beispiel 1, lediglich die Komponenten des Tablettenfilmes variieren.

Die Zusammensetzung je Filmtablette ist in der folgenden Tabelle wiedergegeben:

| **Inhaltsstoff** | **Menge** |
|---|---|
| **Tablettenkern** | |
| Trosfosfamid | 50,000 mg |
| Lactose Monohydrat (Lactose D10) | 50,000 mg |
| Microcrystalline Cellulose (Avicel PH 101) | 30,000 mg |
| Maisstärke | 10,000 mg |
| Talkum | 9,000 mg |
| Colloidal Siliciumdioxid (Aerosil 200 V) | 1,000 mq |
| Σ | 150,000 mg |

| **Film** | |
|---|---|
| Talkum | 2,103 mg |
| Eudragit NE30 D | 1,402 mg |
| Polyethylenglycol 6000 | 0,701 mg |
| Polvsorbat 80 (Tween 80) | 0,140 mg |
| Titandioxid (Pretiox) | 1,402 mg |
| Sicovit Gelb 10 E172 | 0,126 mg |
| Na-Carboxymethylcellulose (Tylopur C30) | 0,122 mg |
| Dimeticon (Silfar SE4) | 0,014 mg |
| Σ | 6,000 mg |

| **Filmtablette** | |
|---|---|
| Σ | 156,000 mg |

Die Herstellung der Filmtabletten erfolgt analog zu der Beschreibung in den Beispielen 1 und 3.

### Beispiel 3: Prozessparameter für einen Ansatz von 30.000 Filmtabletten im Dragierkessel

| | |
|---|---|
| Sprühsystem: | Optima E |
| Dragierkessel-Drehzahl: | 6,5 1/min |
| Kembett-Temperatur: | 25 °C |
| Zulufttemperatur: | 28 °C |
| Sprühfluss: | 10 -15 ml/min |
| Düse: | 0,8 mm |
| Druck: | 3,5 bar |
| Sprüh-Zeit: | 65 min |
| Trocknungszeit: | 5 min |

### Beispiel 4: Trofosfamid-Filmtablette mit einem Cellulosederivat-haltigen Film und einer Dosierung von 100 mg

Die Herstellung der Filmtablette mit einer Dosierung von 100 mg je einzelner Darreichungsform erfolgt analog der Beschreibung in Beispiel 1 und Beispiel 3, wobei die Zusammensetzung in der folgenden Tabelle wiedergegeben ist.

Eine Trofosfamid 100 mg Filmtablette setzt sich wie folgt zusammen:

| **Inhaltsstoff** | **Menge** |
|---|---|
| **Tablettenkern** | |
| Trosfosfamid | 100,000 mg |
| Lactose Monohydrat (Lactose D10) | 20,000 mg |
| Microcrystalline Cellulose (Avicel PH 101) | 15,000 mg |
| Maisstärke | 5,000 mg |
| Talkum | 9,000 mg |
| Colloidal Siliciumdioxid (Aerosil 200 V) | 1,000 mg |
| Σ | 150,000 mg |

| **Film** | |
|---|---|
| Hypromellose (Pharmacoat) | 3,428 mg |
| Polyethylenglycol 6000 | 1,429 mg |
| Titandioxid (Pretiox) | 0,971 mg |
| Sicovit Yellow 10 E172 | 0,143 mg |
| Dimeticon (Silfar SE4) | 0,029 mg |
| Σ | 6,000 mg |

| **Filmtablette** | |
|---|---|
| Σ | 156,000 mg |

### Beispiel 5: Trofosfamid-Filmtablette mit einem Cellulosederivat-haltigen Film und einer Dosierung von 100 mg und einer Tablettenmasse von 200 mg

Eine Trofosfamid Filmtablette setzt sich wie folgt zusammen:

| **Inhaltsstoff** | **Menge** |
|---|---|
| **Tablettenkern** | |
| Trosfosfamid | 100,000 mg |
| Lactose Monohydrat (Lactose D10) | 50,000 mg |
| Microcrystalline Cellulose (Avicel PH 101) | 30,000 mg |
| Maisstärke | 10,000 mg |
| Talkum | 9,000 mg |
| Colloidal Siliciumdioxid (Aerosil 200 V) | 1,000 mg |
| Σ | 200,000 mg |

| **Film** | |
|---|---|
| Hypromellose (Pharmacoat) | 5,142 mg |
| Polyethylenplycol 6000 | 2,144 mg |
| Titandioxid (Pretiox) | 1,456 mq |
| Sicovit Yellow 10 E172 | 0,215 mg |
| Dimeticon (Silfar SE4) | 0,044 mg |
| Σ | 9,000 mg |

| **Filmtablette** | |
|---|---|
| Σ | 209,000 mg |

Die Herstellung der Filmtabletten erfolgt analog der Beschreibung in Beispiel 1 und Beispiel 3.

### Beispiel 6: Lagerstabilität einer Trofosfamid-Filmtablette von Beispiel 1

Bei einer Lagerung von Trofosfamid-Filmtabletten nach Beispiel 1 bei 25 °C wurden folgende Daten ermittelt:

| | Initial | 3 Monate | 6 Monate | 9 Monate | 12 Monate |
|---|---|---|---|---|---|
| Gehalt | 100 % | 100 % | 99 % | 99 % | 100 % |

## Patentansprüche

1. Filmtablette, umfassend einen Trofosfamid enthaltenden Kern und einen durch Aufbringen einer wässrigen, einen oder mehrere Filmbildner enthaltenden Suspension auf den Kern erhaltenen Film.

2. Filmtablette nach Anspruch 1, wobei die Menge an Trofosfamid als pharmazeutisch wirksamer Bestandteil im Bereich von 20 mg bis 200 mg je Filmtablette liegt.

3. Filmtablette nach Anspruch 1 oder 2, welche eine Gesamtmasse im Bereich von 100 mg bis 300 mg aufweist.

4. Filmtablette nach einem der Ansprüche 1 bis 3, wobei der Anteil des Films im Bereich von 1 % bis 15%, bezogen auf die Gesamtmasse der Filmtablette, liegt.

5. Filmtablette nach einem der Ansprüche 1 bis 4, wobei der Anteil des einen oder der mehreren Filmbildner im Bereich von 30% bis 70%, bezogen auf die Gesamtmasse des Films, liegt.

6. Filmtablette nach einem der Ansprüche 1 bis 5, wobei der Filmbildner aus der Gruppe, bestehend aus Cellulosederivaten, Polyvinylderivate und Poly(meth)acryl-säurederivaten, ausgewählt ist.

7. Verfahren zur Herstellung einer in einem der Ansprüche 1 bis 6 definierten Filmtablette, worin die Filmung des Trofosfamid enthaltenden Kerns durch Aufsprühen einer wässrigen Suspension, welche einen oder mehrere Filmbildner enthält, auf dem Kern bei einer Temperatur von 20°C bis 40°C erfolgt.

8. Verfahren nach Anspruch 7, worin die Sprühflussrate zum Aufbringen der wässrigen Suspension im Bereich von 5 bis 25 ml/min liegt.

9. Verfahren nach Anspruch 7 oder 8, wobei die Filmung im Dragierkessel oder mittels Wirbelschichtverfahren, Accela-cota-Verfahren oder Glatt-Coater-Verfahren erfolgt.

## Claims

1. Film-coated tablet, including a trofosfamide containing nucleus and a coating obtained by applying to the nucleus a watery suspension which contains one or several film formers.

2. Film-coated tablet according to claim 1, at which the amount of trofosfamide as pharmaceutically effective agent is within the range of between 20 mg and 200 mg per film-coated tablet.

3. Film-coated tablet according to claims 1 or 2 with a total volume in the range of between 100 mg and 300 mg.

4. Film-coated tablet according to claims 1 through 3, at which the proportion of the film-coating ranges between 1% and 15%, corresponding to the total volume of the film-coated tablet.

5. Film-coated tablet according to claims 1 through 4, at which the proportion of one or several film formers ranges between 30% and 70%, corresponding to the total volume of the film-coating.

6. Film-coated tablet according to claims 1 through 4, at which the film former is selected from the group consisting of cellulose derivatives, polyvinyl derivatives and poly(methyl)acryl acid derivatives.

7. Procedure to produce one of the film-coated tablets defined in claims 1 through 6, at which the film-coating of the trofosfamide containing nucleus is performed by spraying a watery suspension, containing one or several film formers to the nucleus at a temperature of between 20°C and 40°C.

8. Procedure according to claim 7, at which the spraying flow rate to apply the watery suspension ranges between 5 and 25 ml/min.

9. Procedure according to claims 7 or 8, at which the film-coating is performed in the sugar-coating container or by means of the turbulent mixing procedure, Accela-Cota procedure, or Glatt-coating procedure.

## Revendications

1. Comprimé pelliculé, comprenant un noyau contenant un trofosfamide, et une pellicule obtenue sur le noyau par application d'une suspension aqueuse contenant un ou plusieurs agents formant une pellicule.

2. Comprimé pelliculé selon la revendication 1, la quantité de trofosfamide comme composant pharmaceutiquement efficace se situant dans l'intervalle allant de 20 mg à 200 mg par comprimé pelliculé.

3. Comprimé pelliculé selon la revendication 1 ou 2, lequel présente une masse totale dans l'intervalle allant de 100 mg à 300 mg.

4. Comprimé pelliculé selon l'une quelconque des revendications 1 à 3, la proportion de la pellicule étant située dans l'intervalle allant de 1 % à 15 %, rapportée à la masse totale du comprimé pelliculé.

5. Comprimé pelliculé selon l'une quelconque des revendications 1 à 4, la proportion de l'un ou plusieurs agents formant une pellicule étant situés dans l'intervalle allant de 30 % à 70 %, rapportée à la masse totale de la pellicule.

6. Comprimé pelliculé selon l'une quelconque des revendications 1 à 5, l'agent formant une pellicule étant choisi parmi le groupe comprenant des dérivés de la cellulose, des dérivés du polyvinyle et des dérivés d'acides poly(méth)acryliques.

7. Procédé de fabrication d'un comprimé pelliculé défini dans l'une quelconque des revendications 1 à 6, dans lequel le pelliculage du noyau contenant le trofosfamide s'effectue par pulvérisation sur le noyau d'une suspension aqueuse, laquelle contient un ou plusieurs agents formant une pellicule, à une température de 20 °C à 40 °C.

8. Procédé selon la revendication 7, dans lequel le débit de pulvérisation destiné à l'application de la suspension aqueuse se situe dans l'intervalle allant de 5 à 25 ml/min.

9. Procédé selon la revendication 7 ou 8, dans lequel le pelliculage s'effectue dans une cuve de dragéification ou au moyen de procédés à lit fluidisé, du procédé Accela-cota ou du procédé d'enrobage lisse.
